Europäisches Patentamt

**European Patent Office**

**Office européen des brevets**

(11) Publication number: **0 444 194 B1**

# EUROPEAN PATENT SPECIFICATION

(45) Date of publication of patent specification: **22.12.93**

(51) Int. Cl.5: **A61K 33/24**, A61K 33/26, A61K 9/00, A61K 9/10, A61K 47/00, A61N 5/02

(21) Application number: **89909424.7**

(22) Date of filing: **18.08.89**

(86) International application number:
**PCT/JP89/00843**

(87) International publication number:
**WO 90/01939 (08.03.90 90/06)**

The file contains technical information submitted
after the application was filed and not included in
this specification

(54) **AGENT FOR THERMOTHERAPY.**

(30) Priority: **19.08.88 JP 204814/88**

(43) Date of publication of application:
**04.09.91 Bulletin 91/36**

(45) Publication of the grant of the patent:
**22.12.93 Bulletin 93/51**

(84) Designated Contracting States:
**DE FR GB IT**

(56) References cited:
| | |
|---|---|
| **EP-A- 0 186 616** | **EP-A- 0 330 800** |
| **WO-A-85/02772** | **DE-A- 3 443 252** |
| **JP-A-55 160 720** | **JP-A-57 176 474** |
| **JP-A-60 255 728** | **JP-A-63 103 048** |
| **JP-B- 635 379** | **JP-B- 5 913 521** |
| **NL-A- 6 605 491** | **US-A- 4 303 636** |
| **US-A- 4 662 359** | **US-A- 4 735 796** |

**CHEMICAL ABSTRACTS, vol. 109, no. 6, Columbus, OH (US); no. 43353d&NUM;**

(73) Proprietor: **MEITO SANGYO KABUSHIKI KAISHA**
**41, Sasazuka-cho 2-chome**
**Nishi-ku**
**Nagoya-shi Aichi 451(JP)**

(72) Inventor: **TAZAWA, Kenji 7-18,**
**Minamitaikoyama**
**Kosugi-machi**
**Imizu-gun**
**Toyama 939-03(JP)**
Inventor: **NAGAE, Hideo**
**8-7, Higashino-cho 2-chome**
**Kasugai-shi Aichi 486(JP)**

(74) Representative: **Weisert, Annekäte, Dipl.-Ing.**
**Dr.-Ing. et al**
**Patentanwälte**
**Kraus Weisert & Partner**
**Thomas-Wimmer-Ring 15**
**D-80539 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 106, no. 10, Columbus, OH (US); no. 72778q&NUM;

CHEMICAL ABSTRACTS, vol. 85, no. 12, Columbus, OH (US); no. 83193u&NUM;

## Description

This invention relates to a hyperthermic agent comprising a complex of a specified dextran with a magnetic metal or a metal compound. that can be used to treat diseases of man and animals, and particularly to a hyperthermic agent effective for the safe and sure treatment of cancer.

Attempts have recently been made to treat diseases such as malignant tumors or prostatic hypertrophy by warming man or animals systemically or topically. For example, there was proposed a method of treating a malignant tumor which comprises intravenously injecting a suspension of particles of magnetic iron oxide or ferric hydroxide having a diameter not exceeding 1 μm in an aqueous solution of glucose, dextran etc. into a patient, causing it to be taken by cancer cells, exposing the cancer cells to an electromagnetic field to treat the particles by induction heating and thus causing the cancer cells to die (see Japanese Patent Publication No. 5379/1988).

However, this suspension of magnetic particles has poor colloidal stability and when it encounters a body fluid such as blood or a tissue fluid, it aggregates within a short period of time. In intravenous administration, the magnetic particles deposit on the blood vessels or tissues before they reach the cancer cells, and it is difficult to concentrate them on the cancer cells. Furthermore, the dose of the magnetic particles is limited because the aggregated particles will induce circulation disorder.

To avoid this difficulty, direct injection of the magnetic particle suspension into a tumor tissue was investigated [See Abstracts of Papers in the 44th Annual Meeting of the Japanese Cancer Association, Paper No. 1800 (1985)]. According to this method, the magnetic particles readily aggregate within the tumor tissues and cannot be uniformly distributed. Accordingly, the tumor cells do not completely die, and there is a risk of repalse and metastasis. The effect of the magnetic particles is therefore uncertain. Another defect is that the localisation of the magnetic particles sometimes causes the formation of an abnormally high temperature portion and will induce damage of a normal tissue, particularly the blood vessels.

As another problem, these magnetic particles have very poor metabolizability in vivo. When the treatment of tumor is successful and the patient survives for a long period of time, the unknown toxicity of the magnetic particles is feared. Also, these magnetic particles are likely to constitute a great obstacle to the prognosis of cancer by a magnetic resonance imaging device which recently came into widespread use.

Earlier European application EP-A-0 330 800 published on September 6, 1989, has the filing date of March 4, 1988.

Its content as filed is therefore considered, pursuant to Article 54(3) and (4) EPC, as comprised in the state of the art relevant to the present application. In this earlier patent application, nothing is, however, said about the special properties of the dextran as used in this invention.

WO-A-85/02772, DE-A-34 43 252 and EP-A-0 186 616 relate to complexes of dextran and metal compounds and compositions comprising them. However, in none of these documents the dextran-magnetic metal or metal compound complex as used in the present invention for such a specific use as in hyperthermic therapy is taught or suggested.

It is an object of this invention to provide a hyperthermic agent which is free from the aforesaid defects and has a very high induction heating efficiency, excellent colloidal stability in vivo, low toxicity and good metabolizability in vivo.

This invention relates to a hyperthermic agent comprising a complex of dextran having a weight average molecular weight of 1,000 to 100,000 with a magnetic metal or metal compound having an average particle diameter in the range of 5.0 to 15.0 nm (50 to 150 Å) as an active ingredient.

The complex of dextran with a magnetic metal or metal compound used as the active ingredient of the hyperthermic agent of this invention is produced by chemically reacting dextran with the magnetic metal or metal compound. This complex clearly distinguishes from the composition described in the above-cited Japanese Patent Publication No. 5379/1988 which is a mere suspension of the magnetic particles in an aqueous dextran solution.

Dextran used for preparation of the complex may be any of those known dextran products. Those having a weight average molecular weight of 1,000 to 100,000, preferably 4,000 to 10,000, are used in view of colloidal stability and a temperature elevating effect. The dextran may be unmodified. It is possible and preferable, however, to use modified products of dextran obtained by known methods, for example, dextran modified at its reducing ends by treatment with an alkali, a halogen or halogenous acid; and dextran modified at its reducing ends obtained by treatment with a cyanide ion and subseqeunt hydrolysis (for the modifying method, see, for example, Japanese Patent Publications Nos. 5557/1970 and 13521/1984 and Japanese Laid-Open Patent Publication No. 233001/1986). Accordingly, the term "dextran", as used in the present specification and claims, denotes not only unmodified dextran but also modified dextran.

The magnetic metal or metal compound to be reacted with dextran is a magnetic metal or metal compound which generates heat upon induction heating, and includes ferromagnetic and superparamagnetic materials. These magnetic materials are generally used in the form of fine particles having an average particle diameter of usually 3.0-50.0 nm (30-500 Å), preferably 5.0-15.0 nm (50-150 Å).

Examples of the magnetic metal are transition metals such as iron, nickel, cobalt and gadolinium, and iron is especially preferred. Examples of the magnetic metal compounds are oxides of transition metals such as iron, nickel and cobalt, and ferrite. Triiron tetraoxide and gamma-iron oxide are preferred.

The reaction of dextran with the magnetic metal or metal compound can be carried out, for example, in accordance with the method described in Japanese Patent Publication No. 13521/1984, by adding dextran or its aqueous solution to an aqueous sol of the magnetic metal or metal compound and heating the mixture at a temperature of about 90°C to the refluxing temperature for about 20 to 120 minutes under neutral or weakly acidic conditions.

The complex may also be prepared, for example, by the method described in U. S. Patent No. 4,101,435, by producing a magnetic metal compound such as magnetic iron oxide in an aqueous medium in the presence of dextran, preferably modified dextran obtained by alkali treatment, and then heating it at a temperature of about 90°C to the refluxing temperature for about 30 to 120 minutes under neutral or weakly acidic conditions.

The complex of dextran with the magnetic metal or metal compound so prepared is formulated in a form suitable for administration as a hyperthermic agent. The route of administration may generally be injection or instillation into vein, tumor tissue, artery, and urinary bladder, etc. If it is desirable, it may be administered orally or intrarectally.

For administration by injection or instillation, the above complex may be dissolved in distilled water for injection or physiological saline solution in a concentraion of usually 1 to 60 % (w/v), preferably 5 to 20 % (w/v), in accordance with the method customarily used in the field of pharmaceutical science. As additives, various physiologically acceptable aids may be properly incorporated. Examples thereof are inorganic salts such as sodium chloride, monosaccharides such as dextrose, sugar alcohols such as mannitol and sorbitol, organic acid salts such as citrates and tartrates, a phosphoric acid buffer and a tris buffer.

For oral and intrarectal administration, the above complex may be formulated together with suitable preparation aids into tablets, granules, capsules, syrups, powders or suppository in accordance with a customary method in the field of pharmaceutical science.

The dose of the hyperthermic agent of this invention may vary depending upon the severity, age and the site of the lesion of a patient to be treated, but it should be administered in such a dose that it can be maintained in tissues or body fluids in concentrations at least capable of overcoming the cooling ability of the body. The concentration will be about 0.5-5.0 % (w/v), for example, when it is to be administered to the inside of the malignant tumor, the suitable dose is 1 to 50 mg, calculated as metal, per $cm^3$ of the volume of the malignant tumor. This, however, is a tentative criterion, and based on the diagnosis of a physician, it may be administered in smaller or larger doses.

The patient to which the hyperthermic agent of this invention is administered is placed in a high frequency magnetic field of an induction heating device and treated while the temperature is monitored. The suitable frequency in the induction heating device is generally 20 KHz to 10 MHz, preferably 50 to 500 KHz. The temperature monitoring can be carried out, for example, by using a copper-platinum type sensor coated with a vinyl chloride resin which is used in a Model TM 54 machine made by Baily Co., of U. S. A.

The hyperthermic agent provided by this invention uses a complex obtained by chemical reaction of dextran with the magnetic metal or metal compound. Accordingly, its stability in a colloidal form is superior and it can be uniformly dispersed in a specific tissue in the body. Thus, the hyperthermic agent of this invention has an excellent hyperthermic efficiency, low toxicity and good metabolizability, and can be used widely in hyperthermic therapy of malignant tumors (cancer), prostatic hypertrophy, wounds, etc.

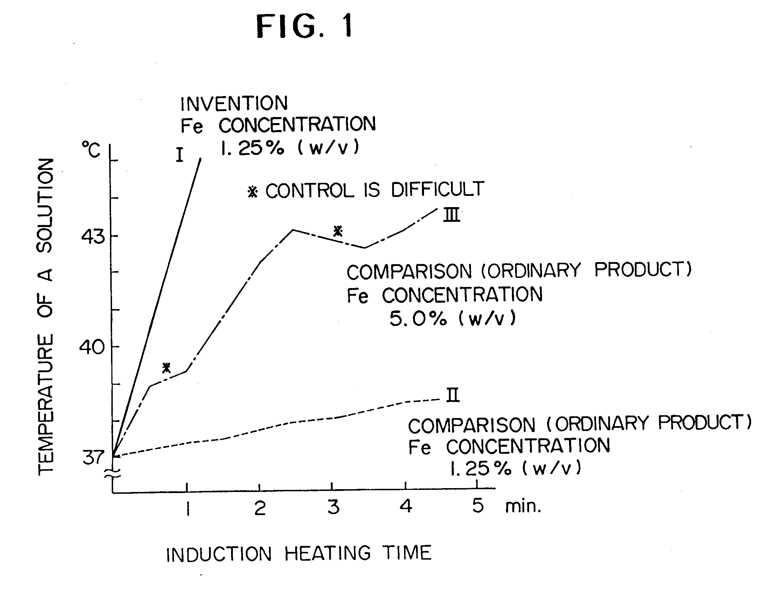

Figure 1 is a graph showing results of an induction heating test in Test Example 1.

The following Referential Example, Examples and Test Examples illustrate the present invention more specifically.

REFERENTIAL EXAMPLE 1

Production of a complex

Alkali-treated modified dextran (169 g) having a weight average molecular weight of 4,000 was dissolved in 552 ml of water. While the solution was heated over a water bath, 228 ml of an aqueous solution of a mixture of 22.28 g of ferrous chloride tetrahydrate and 37.02 g of ferric chloride and 400 ml of 3N sodium hydroxide were added. The solution was neutralized with hydrochloric acid, and heated under reflux for 1 hour, followed by cooling. Coarse particles were removed by centrifugal separation, and an equal amount of methanol was added to the supernatant to obtain a crude complex of dextran with magnetic iron oxide as a precipitate. The precipitate was dissolved in water, and the solution was dialyzed overnight against running water. The dialyzate was adjusted to pH 8, concentrated by an evaporator, then filtered through a 0.20 $\mu$m filter, and lyophilized to give a powder (black powder, 35 g) of the complex of dextran with magnetic iron oxide. The properties of the resulting magnetic iron oxide-dextran complex are shown in Table 1.

Table 1

| | |
|---|---|
| Iron content | 46.70 % |
| Dextran content | 27.71 % |
| Dextran/iron weight ratio | 0.59 |
| Average particle diameter of the magnetic iron oxide portion | 7.7 nm (77 Å) |
| Coercivity | 240 A/m (3 Oersted) |
| Susceptibility | 0.295 $(gFe)^{-1}$ |
| Saturation magnetization | 0.103 $Wb/m^2 \cdot gFe$ (82.3 emu/gFe) |
| $T_2$ relaxivity | 224 $liter.mmole^{-1}.sec^{-1}$ |

REFERENTIAL EXAMPLE 2

Dextran (250 g) having a weight average molecular weight of 7,000 was dissolved in 1.0 liter of water, and 25 g of iodine ($I_2$) was added thereto. Under stirring, 30 ml of 40 % NaOH was added for about 1 hour, and the mixture was further stirred for 2 hours. To the reaction solution was added methanol in an amount which was three times that of the reaction solution to obtain a precipitate. A reprecipitation procedure of redissolving the precipitate in water and adding methanol in an amount of three times that of the reaction solution was further repeated twice. An aqueous solution of the precipitate was filtered, and the filtrate was adjusted to pH 7.5, concentrated under reduced pressure and then lyophilized to give modified dextran (white powder, 225 g).

REFERENTIAL EXAMPLE 3

Dextran (272 g) having a weight average molecular weight of 4,000 was dissolved in 1.0 liter of water. While keeping pH at 3.0±0.1 with conc. hydrochloric acid, 164 g of sodium hypochlorite ($NaClO_2$) was added for about 1 hour under stirring. Further, while keeping pH at 3.0±0.1, stirring continued for 3 hours and then for 15 hours. To the reaction solution was added methanol in an amount which was 3.5 times that of the reaction solution to obtain a precipitate. A reprecipitation procedure of redissolving the precipitate in water and adding methanol in an amount of 3.5 times that of the reaction solution was repeated twice. An aqueous solution of the precipitate was filtered, and the filtrate was adjusted to pH 7.5, concentrated under reduced pressure, and then lyophilized to give modified dextran (pale yellow powder, 218 g).

REFERENTIAL EXAMPLE 4

A mixture of 1.0 liter of a 1M ferric chloride aqueous solution and 500 ml of a 1M ferrous chloride aqueous solution was added with stirring to a slurry obtained by adding water to 5 liters of Amberlit® IRA-410 (an ion exchange resin made by Rohm & Haas Co.) such that the total amount became 7 liters, while keeping pH at 8.0 to 8.7. To the reaction solution was immediately added conc. hydrochloric acid to adjust pH to 1.6. At the same pH, stirring continued for 1 hour, and the ion exchange resin was separated by filtration. While adding water to the resulting sol, ultrafiltration was conducted to obtain 2.3 liters of a magnetic iron oxide aqueous sol (pH 2.8, Fe content 26 mg/ml). Six hundred milliliters of the sol was mixed with 300 ml of a 25 w/v % aqueous solution of the modified dextran obtained in Referential Example 2, and the mixture was heated under reflux for 1 hour. After cooling, methanol was added to the reaction solution until the concentration reached 47 %, and the resulting precipitate was dissolved in water and dialyzed overnight against running water. After pH was adjusted to 8, concentration under reduced pressure, filtration and lyophilization were carried out to obtain a magnetic iron oxide-dextran complex (black powder, 37 g).

The properties of the complex are shown in Table 2.

Table 2

| Iron content | 39.3 % |
|---|---|
| Dextran content | 41.0 % |
| Dextran/iron weight ratio | 1.04 |
| Average particle diameter of the magnetic iron oxide portion | 8.0 nm (80 Å) |
| Coercivity | 320 A/m (4 Oersted) |
| Susceptibility | 0.27 $(gFe)^{-1}$ |
| Saturation magnetization | 0.113 $Wb/m^2 \cdot gFe$ (90.1 emu/gFe) |
| $T_2$ relaxivity | 272 $liter.mmole^{-1}.sec^{-1}$ |

REFERENTIAL EXAMPLE 5

Six hundred milliliters of the magnetic iron oxide aqueous sol produced in Referential Example 4 was mixed with 300 ml of a 25 % (w/v) aqueous solution of the modified dextran obtained in Referential Example 3, and the mixture was then treated as in Referential Example 4 to obtain a magnetic iron oxide-dextran complex (black powder, 33 g). The properties of the complex are shown in Table 3.

Table 3

| Iron content | 45.8 % |
|---|---|
| Dextran content | 32.0 % |
| Dextran/iron weight ratio | 0.70 |
| Average particle diameter of the magnetic iron oxide portion | 7.8 nm (78 Å) |
| Coercivity | 320 A/m (4 Oersted) |
| Susceptibility | 0.26 $(gFe)^{-1}$ |
| Saturation magnetization | 0.113 $Wb/m^2 \cdot gFe$ (89.9 emu/gFe) |
| $T_2$ relaxivity | 284 $liter.mmole^{-1}.sec^{-1}$ |

EXAMPLE 1

Physiological saline was added to 2170 mg of the magnetic iron oxide-dextran complex powder obtained in Referential Example 1 to prepare 10 ml of a solution. In a water bath, the solution was heated to 50 °C, and filtered and sterilized by filtering through a Millipore filter having a pore size of 0.22 micrometer. It was then sealed up in a sterilized glass ampoule.

EXAMPLE 2

Mannitol (200 mg) was added to 2170 mg of the magnetic iron oxide-dextran complex powder obtained in Referential Example 1. Physiological saline was added to form 10 ml of a solution. It was sealed in a glass ampoule and sterilized in an autoclave at 121 °C for 20 minutes.

EXAMPLE 3

Mannitol (200 mg ) and 2170 mg of the magnetic iron oxide-dextran complex powder obtained in Referential Example 1 were well mixed, and physiological saline was added in use to form 10 ml of a solution. It was sterilized by filtering through a Millipore filter having a pore size of 0.22 micrometer.

EXAMPLE 4

To 3,053 mg of the magnetic iron oxide-dextran complex powder obtained in Referential Example 4 was added 127 mg of disodium citrate, and the mixture was dissolved in distilled water for injection to form 100 ml of a solution. The solution was heated in a water bath to 50°C, and filtered and sterilized by using a Millipore filter having a pore size of 0.22 micrometer. It was sealed up in a sterilized vial.

EXAMPLE 5

To 2,620 mg of the magnetic iron oxide-dextran complex powder obtained in Referential Example 5 was added physiological saline to form 100 ml of a solution. The solution was formed into a preparation in the same way as in Example 1.

TEST EXAMPLE 1

Distilled water for injection was added to the magnetic iron oxide-dextran complex obtained in Referential Example 1 to form 5 ml of a solution having an Fe concentration of 1.25 % (w/v). It was put in a petri dish having a diameter of 3 cm, and subjected to an induction heating test at a frequency of 500 KHz with an output of 4.6 KW using an induction heating device (model TY made by Yamamoto Vinyter Co., Ltd., JAPAN). As a comparative agent, suspensions of magnetic iron oxide having an average particle diameter of about 8.0 nm (80 Å) in a 10 % (w/v) aqueous solution of dextran (weight average molecular weight 70,000) with an Fe concentration of 1.25 % (w/v) and 5.0 % (w/v) respectively were prepared in an amount of 5 ml each. These suspensions were subjected to an induction heating test under the same conditions as above.

The temperature of the solution was measured by a temperature measuring device (model TM54 made by Baily Co., U. S. A. ).

The agent of this invention showed a temperature rise of about 7 °C by induction heating for 1 minutes. On the other hand, the comparative agent in the same Fe concentration showed a temperature increase of only 0.4 °C by induction heating under the same conditions. The comparative agent in an Fe concentration of 5.0 % (w/v) prepared to increase the temperature elevating efficiency certainly showed an increased temperature elevation efficiency but was liable to form sedimented matter. When electricity was continued to be applied under the same conditions, the temperature rise was irregular. The results of the induction heating test are shown in the graph of Figure 1.

TEST EXAMPLE 2

Five milliliters of the injection (this invention) obtained in Example 4 was charged in a petri dish having a diameter of 3 cm and subjected to an induction heating test with an output of 1.4 KW using the same device as in Test Example 1.

The same comparative agent as in Test Example 1 was used and subjected to the same test as in Test Example 1.

The results shown in Table 4 were obtained.

Table 4

| Test solution | Temperature rise (°C/min) |
|---|---|
| Invention | 2.2 |
| Comparison | 0.2 |

TEST EXAMPLE 3

Five milliliters of the injection (this invention) obtained in Example 5 was used and subjected to the same induction heating test as in Test Exmaple 2.

The results shown in Table 5 were obtained.

Table 5

| Test solution | Temperature rise (°C/min) |
|---|---|
| Invention | 2.4 |
| Comparison | 0.2 |

TEST EXAMPLE 4

The relation of the physical and chemical properties of the agents of this invention to temperature rise by induction heating was examined under the same conditions as in Test Example 2. The results shown in Table 6 were obtained.

Table 6

| Items examined and conditions | | Temperature rise (°C/min.) | Remarks |
|---|---|---|---|
| Weight average molecular weight of starting dextran | 4,000 | 4.5 | The iron oxide portion had a particle diameter of about 8.0 nm (80 Å) in all runs. |
| | 7,000 | 4.2 | |
| | 20,000 | 2.1 | |
| | 70,000 | 1.5 | The test solution had an Fe concentration of 2.5 % (w/v) in all runs. |
| Average particle diameter of the iron oxide portion | 3.0 nm (30 Å) | 0.4 | Dexran had a molecular weight of 4,000 in all runs. |
| | 6.0 nm (60 Å) | 2.1 | |
| | 10 nm (100 Å) | 4.3 | The test solution had an Fe concentration of 2.5 % (w/v) in all runs. |
| Susceptibility | 1.01 (gFe)$^{-1}$ | 1.5 | The test solution had an Fe concentration of 2.5 % (w/v) in all runs. |
| | 1.08 (gFe)$^{-1}$ | 2.1 | |
| | 2.95 (gFe)$^{-1}$ | 4.5 | |
| Concentration of iron oxide (as Fe) | 1.25 % (w/v) | 2.6 | Dextran had a molecular weight of 4,000 in all runs. |
| | 2.5 % (w/v) | 5.0 | |
| | 5.0 % (w/v) | 9.1 | The iron oxide portion had a particle diameter of about 8.0 nm (80 Å). |

A negative correlation was seen between the temperature elevation rate and the molecular weight of dextran, and a positive correlation was seen among the average particle diameter of the iron oxide portion, the susceptibility and the molecular weight of dextran.

TEST EXAMPLE 5

A solution having an Fe concentration of 10 % (w/v) was prepared from the magnetic iron oxide-dextran complex obtained in Referential Example 1 and distilled water for injection. One milliliter of the solution was mixed with 1 ml of fresh rabbit serum. The mixture was maintained at 37°C, and observed with naked eyes for aggregation. As a comparative agent, a suspension having an Fe concentration of 10 % (w/v) was prepared by suspending magnetic iron oxide particles having an average particle diameter of about 8.0 nm (80 Å) in a 10 % (w/v) aqueous solution of dextran having a weight average molecular weight of 70,000 was used, and tested in the same way as above. The results are shown in Table 7.

Table 7

| Test solution | Time elapsed until aggregation occurred (minutes) |
|---|---|
| Invention | >1,440 |
| Comparison | <1 |

The agent of the invention had superior colloidal stability in the serum.

TEST EXAMPLE 6

One milliliter of distilled water for injection was added to 217 mg of the magnetic iron oxide-dextran complex obtained in Referential Example 1. The solution was sterilized by filtration, and then injected into a tumor at a rate of 0.10 ml per $cm^3$ of the volume of the tumor. Twenty-four hours later, the state of distribution of the complex was examined. Specifically, a glass plate for colony calculation with a pattern of squares each side measuring 3 mm was applied to the surface of the tumor, and the ratio of parts where the black injected solution was present to the remainder was determined.

As a comparative agent, a suspension having an Fe concentration of 10 % (w/v) was prepared by suspending magnetic iron oxide having an average particle diameter of about 8.0 nm (80 Å) in a 10 % (w/v) aqueous solution of dextran having a weight average molecular weight of 70,000, and tested as described above. The suspension was blackish brown.

The results are shown in Table 8.

Table 8

| Test solution | Distribution rate (%)<br>(the area of parts colored with the test solution/total area of the surface) x 100 |
|---|---|
| Invention<br>Comparison | 91<br>38 |

The distribution of the agent of this invention in the tumor was uniform.

TEST EXAMPLE 7

Meth-A fibrosarcoma cells were transplanted into the abdominal cavity of BALB/c female mice at a rate of $1 \times 10^5$, and on the 8th day from the day of transplantation, a solution of the magnetic iron oxide-dextran complex obtained in Referential Example 1 was intraperitoneally administered to the mice at a dose of 24 mg/mouse as Fe. Two hours later, induction heating was started at a frequency of 500 KHz with an output of 4.6 KW using the same induction heating device as used in Test Example 1. The current was passed through the device intermittently so that the temperature measured of the rectum reached 40.5 to 41.5 °C. The hyperthermic therapy was carried out for 10 minutes, and the number of days during which the mice survived was determined. As a control group, the above solution was intraperitoneally injected, but the mice were not subjected to induction heating. The increased in life span (ILS) was calculated in accordance with the following equation. Seven mice were used per group.

$$ILS (\%) = (HT/C-1) \times 100$$

where HT is the average days of survival of the group treated by hyperthermia, and C is the average days of survival of the control group.

As a comparative agent, a suspension of triiron tetraoxide having an average particle diameter of 100.0 nm (1,000 Å) in a 5 % aqueous solution of glucose with an iron oxide concentration of 5 % (w/v) was prepared and intraperitoneally injected at a rate of 24 mg as iron per mouse, and tested in the same manner as above.

The results obtained are shown in Table 9.

Table 9

| Test solution | ILS (%) |
|---|---|
| Invention | 120 |
| Comparison | -25 |

The tumor in the mice used in this test example corresponded to cancer in the last stage. Under these conditions, the agent of this invention showed an appreciable life prolonging effect.

On the other hand, no life prolonging effect was observed in the comparison.

TEST EXAMPLE 8

Meth-A fibrosarcoma cells were transplanted subcutaneously in the inguinal portion of BALB/c mice at a rate of $1 \times 10^6$ cells per mouse to form a solid tumor. When the long diameter of this tumor reached 1 to 1.5 cm, an aqueous solution of the complex obtained in Referential Example 1 was injected into the tumor in an amount of 12 mg as iron per $cm^3$ of the volume of the tumor calculated from the following equation. Two hours later, induction heating was started at a frequency of 500 KHz with an output of 4.6 KW by using the same induction heating device as used in Test Example 1. The current of the induction heating device was passed intermittently so that the temperature of the skin at the injected site, measured by the same temperature measuring device as use in Test Example 7, was maintained at 41 to 43 °C for 10 minutes.

For comparison, the same comparative agent as used in Test Example 6 was injected into the tumor of the mice in the same amount as iron, and the injected portion was induction-heated under the same conditions as above.

Thereafter, the number of days of survival was determined on these tumor-bearing mice treated as above, and those which were treated by injection as above but were not subjected to induction heating. By the same equation as in Test Example 5, the ILS (%) was calculated.

$$\text{Tumor volume } (cm^3) = \frac{\pi \, d_1 d_2^2}{6}$$

wherein $d_1$ is the long diameter (cm) of the tumor, and $d_2$ is the short diameter (cm) of the tumor.

The results obtained are shown in Table 10.

Table 10

| Test solution | ILS (%) |
|---|---|
| Invention | 87 |
| Comparison | 25 |

TEST EXAMPLE 9

Ascites liver cancer cells, AH60C cells were transplanted intraperitoneally to Donryu-strain rats at a rate of $1 \times 10^6$ cells per rat, and six days later, a 2.6 % (w/v) aqueous solution [1.2 % (w/v) calculated as Fe] or a 5.2 % (w/v) aqueous solution of the complex obtained in Referential Example 1 was prepared and injected intraperitoneally to the cancer-bearing rats at a dose of 5 ml per rat. About 2 hours later, induction heating was carried out by the same method as in Test Example 7. Seven rats which were half of the total number of rats in the group to which 5 ml of the 5.2 % (w/v) aqueous solution had been injected were treated by induction heating under the same conditions on the day after the next day.

The number of days of survival was then determined, and the ILS (%) was calculated as in Test Example 7.

The results are shown in Table 11.

Table 11

| Test solution (dose: mg Fe/body) | | Induction heating | ILS (%) |
|---|---|---|---|
| Invention | 60 | once | 156 |
| Invention | 120 | once | 157 |
| Invention | 120 | twice | 236 |
| Comparison | 120 | once | 0 |
| Comparison | 120 | twice | 51 |

TEST EXAMPLE 10

Ascites liver cancer cells, AH60C cells were transplanted intraperitoneally to Donryu-strain rats at a rate of $5 \times 10^6$ cells per rat, and six days later, the solution for injection of this invention obtained in Example 4 was intraperitoneally injected to the cancer-bearing rats at a dose of 5 ml per rat. The effect of the antitumor activity was tested as in Test Example 7. The results shown in Table 12 were then obtained.

Table 12

| Test solution | Dose (mg Fe/body) | ILS (%) |
|---|---|---|
| Invention | 60 | 161 |
| Comparison | 60 | 3 |

TEST EXAMPLE 11

Using the solution for injection of this invention obtained in Example 5, the effect of the antitumor activity was tested as in Test Example 10. The results shown in Table 13 were then obtained.

Table 13

| Test solution | Dose (mg Fe/body) | ILS (%) |
|---|---|---|
| Invention | 60 | 158 |
| Comparison | 60 | -1 |

TEST EXAMPLE 12

Ascites liver cancer, AH60C cells were transplanted subcutaneously into the foot pad of rats of the same strain as used in Test Example 9 at a rate of $1 \times 10^6$ cells per rat to form a solid tumor. When the thickness of the foot pad exceeded 1 cm and the tumor was observed entirely in the foot, the volume of the foot into which the cancer cells had been transplanted was measured by the water-replacing method. The complex obtained in Example 1 was injected into the tumor in an amount of 12 mg as iron per $cm^3$ of the foot volume. About two hours later, the same induction heating treatment as in Test Example 8 was carried out, and 12 days later, the foot volume was again measured.

The results shown in Table 14 were obtained.

Table 14

| Test solution | Foot volume ($cm^3$) | |
| --- | --- | --- |
| | 0 day | 12 days |
| Invention | 2.6 | 2.0 |
| Comparison | 2.4 | 2.5 |
| Negative control | 1.8 | 3.0 |

TEST EXAMPLE 13

A saline solution of the complex obtained in Referential Example 1 (iron concentration 0.3 % w/v) and a 10 % (w/v) aqueous diluted solution of dextran (weight average molecular weight 70,000) and iron oxide (iron concentration 0.3 % w/v) used as the comparative agent in Test Example 6 were injected intraperitoneally into normal BALB/c mice, seven per group, in an amount of 5 ml each. The mice were then treated by induction heating continuously for 10 minutes at a frequency of 500 KHz with an output of 4.6 KW by using the same device as used in Test Example 7. The side effect was evaluated by the survival of the mice up to 2 weeks later.

The results shown in Table 15 were obtained.

Table 15

| Test solution | Mortality (number of dead animals/number of animals treated) |
| --- | --- |
| Invention | 0/5 |
| Comparison | 5/5 |

Pathological autopsy showed that when the comparative agent was administered, marked aggregation and deposition of the iron oxide particles occurred between the liver and the stomach and part of the mesentery. This means that during induction heating, an abnormally high temperature was generated at the site where the aggregation was seen and, it was not reflected in the temperature of the rectum.

On the other hand, the agent of this invention uniformly distributed, and caused little side-effect.

TEST EXAMPLE 14

Using dd-strain mice, five per group, mortality in intravenous administration of a saline solution of the complex obtained in Referential Example 1 was measured, and $LD_{50}$ value was calculated by Litchfield & Wilcoxon method. The $LD_{50}$ value of the comparative agent used in Test Example 6 was measured in the same.

The results shown in Table 16 were obtained.

Table 16

| Test solution | $LD_{50}$ [95% confidence limit] (mg Fe/kg) |
| --- | --- |
| Invention (100 mg/ml as Fe) | 2,400 [1,600 - 3,600] |
| Comparison (ditto) | 220 (169 - 286) |

The agent of this invention has extremely low toxicity.

TEST EXAMPLE 15

The agent of this invention and the comparative agent used in Test Example 14 were administered intravenously to Wistar-strain rats in an amount of 5 mg/kg as Fe. The liver was excised periodically and

12

homogenized to form homogenates. The metabolizability of the test agents in vivo were estimated using $T_2$ relaxivity in the homogenates of liver, which was measured by NMR analyser.

The half life in the liver was as shown in Table 17.

Table 17

| Test solution | Half life (days) in the liver |
|---|---|
| Invention | 3.1 |
| Comparison | >7 |

INDUSTRIAL UTILIZABILITY

As has been stated above, the hyperthermic agent of this invention is excellent in colloidal stability, good in uniform dispersibility in specific tissues in vivo, therefore excellent in hyperthermic effect, low in toxicity and good in metabolizability; and it can widely be used in hyperthermic therapy of malignant tumors (cancers), prostatic hypertrophy or wounds.

**Claims**

1. A hyperthermic agent comprising a complex of dextran having a weight average molecular weight of 1,000 to 100,000 with a magnetic metal or metal compound having an average particle diameter in the range of 5.0 to 15.0 nm (50 to 150 Å) as an active ingredient.

2. The hyperthermic agent of claim 1 in which the dextran has a weight average molecular weight of 4,000 to 10,000.

3. The hyperthermic agent of claim 1 in which the dextran is modified dextran obtained by treatment with an alkali, a halogen or a halogenous acid to modify its reducing ends, or modified dextran obtained by treatment with a cyanide ion and subsequent hydrolysis to modify its reducing ends.

4. The hyperthermic agent of claim 1 in which the magnetic metal or metal compound is ferromagnetic or superparamagnetic.

5. The hyperthermic agent of claim 1 in which the magnetic metal is metallic iron, metallic nickel, metallic cobalt or metallic gadolinium.

6. The hyperthermic agent of claim 1 in which the magnetic metal compound is an oxide of iron, nickel or cobalt.

7. The hyperthermic agent of claim 6 in which the magnetic metal compound is triiron tetraoxide or gamma iron oxide.

8. The hyperthermic agent of claim 1 for use in hyperthermic treatment of a malignant tumor in man and animals.

9. A composition of a hyperthermic agent comprising an effective amount of a complex of dextran having a weight average molecular weight of 1,000 to 100,000 with a magnetic metal or metal compound having an average particle diameter in the range of 5.0 to 15.0 nm (50 to 150 Å) and a pharmaceutically acceptable aid.

10. The composition of the hyperthermic agent of claim 9 which is in the form of an injection or an instillation.

11. A hyperthermic agent according to one of claims 1 to 8 or a composition according to claim 9 or 10 for use in hyperthermic therapy.

**Patentansprüche**

1. Hyperthermisches Mittel, dadurch **gekennzeichnet,** daß es einen Komplex aus Dextran mit einem gewichtsdurchschnittlichen Molekulargewicht von 1000 bis 100.000 mit einem magnestischen Metall oder einer Metallverbindung mit einem durchschnittlichen Teilchendurchmesser im Bereich von 5,0 bis 15,0 nm (50 bis 150 Å) als aktiven Bestandteil umfaßt.

2. Hyperthermisches Mittel nach Anspruch 1, dadurch **gekennzeichnet,** daß das Dextran ein gewichtsdurchschnittliches Molekulargewicht von 4.000 bis 10.000 besitzt.

3. Hyperthermisches Mittel nach Anspruch 1, dadurch **gekennzeichnet,** daß das Dextran modifiziertes Dextran, das durch Behandlung mit einem Alkali, einem Halogen oder einer Salz-bildenden bzw. Halogenidbildenden Säure zur Modifizierung seiner reduzierenden Enden erhalten worden ist, oder modifiziertes Dextran, das durch Behandlung mit Cyanidionen und anschließender Hydrolyse zur Modifizierung seiner reduzierenden Enden erhalten worden ist, ist.

4. Hyperthermisches Mittel nach Anspruch 1, dadurch **gekennzeichnet,** daß das magnetische Metall oder die Metallverbindung ferromagnetisch oder superparamagnetisch ist.

5. Hyperthermisches Mittel nach Anspruch 1, dadurch **gekennzeichnet,** daß das magnetische Metall metallisches Eisen, metallisches Nickel, metallisches Cobalt oder metallisches Gadolinium ist.

6. Hyperthermisches Mittel nach Anspruch 1, dadurch **gekennzeichnet,** daß die magnetische Metallverbindung ein Oxid des Eisens, des Nickels oder des Cobalts ist.

7. Hyperthermisches Mittel nach Anspruch 6, dadurch **gekennzeichnet,** daß die magnetische Metallverbindung Trieisentetraoxid oder gamma-Eisenoxid ist.

8. Hyperthermisches Mittel nach Anspruch 1 für die Verwendung bei der hyperthermischen Behandlung maligner Tumore bei Menschen und Tieren.

9. Zusammensetzung aus einem hyperthermischen Mittel, dadurch **gekennzeichnet,** daß sie eine wirksame Menge eines Komplexes aus Dextran mit einem gewichtsdurchschnittlichen Molekulargewicht von 1.000 bis 100.000 mit einem magnetischen Metall oder einer Metallverbindung mit einem durchschnittlichen Teilchendurchmesser im Bereich von 5,0 bis 15,0 nm (50 bis 150 Å) und ein pharmazeutisch annehmbares Hilfsmittel enthält.

10. Zusammensetzung des hyperthermischen Mittels nach Anspruch 9, dadurch **gekennzeichnet,** daß es in einer für die Injektion oder Instillation geeigneten Form vorliegt.

11. Hyperthermisches Mittel nach einem der Ansprüche 1 bis 8 oder eine Zusammensetzung nach Anspruch 9 oder 10 für die Verwendung in der hyperthermischen Therapie.

**Revendications**

1. Agent hyperthermique comprenant un complexe d'un dextrane ayant une masse moléculaire moyenne de 1000 à 100.000 avec un métal ou un composé métallique magnétique présentant un diamètre moyen de particule de l'ordre de 5,0 à 15,0 nm (50 à 150 Å) comme ingrédient actif.

2. L'agent hyperthermique de la revendication 1 dans lequel le dextrane a une masse moléculaire moyenne de 4.000 à 10.000.

3. L'agent hyperthermique de la revendication 1 dans lequel le dextrane est un dextrane modifié obtenu par traitement avec une base, un halogène ou un acide halogéné pour modifier ses extrémités réductrices, ou un dextrane modifié obtenu par traitement avec un ion cyanure et hydrolyse ultérieure pour modifier ses extrémités réductrices.

**4.** L'agent hyperthermique de la revendication 1 dans lequel le métal ou composé métallique magnétique est ferromagnétique ou superparamagnétique.

**5.** L'agent hyperthermique de la revendication 1 dans lequel le métal magnétique est le fer métallique, le nickel métallique, le cobalt métallique ou le gadolinium métallique.

**6.** L'agent hyperthermique de la revendication 1 dans lequel le composé métallique magnétique est un oxyde de fer, de nickel ou de cobalt.

**7.** L'agent hyperthermique de la revendication 6 dans lequel le composé métallique magnétique est le tétraoxyde de fer triple ($Fe_3O_4$) ou l'oxyde de fer-gamma.

**8.** L'agent hyperthermique de la revendication 1 en vue d'une utilisation pour un traitement hyperthermique d'une tumeur maligne de l'homme ou des animaux.

**9.** Une composition d'un agent hyperthermique comprenant une quantité efficace d'un complexe d'un dextrane ayant une masse moléculaire moyenne de 1000 à 100.000 avec un métal ou un composé métallique magnétique présentant un diamètre moyen de particule de l'ordre de 5,0 à 15,0 nm (50 à 150 Å) et un excipient pharmaceutiquement acceptable.

**10.** La composition de l'agent hyperthermique de la revendication 9 qui est sous la forme d'une injection ou d'une instillation.

**11.** Un agent hyperthermique selon l'une des revendications 1 à 8 ou une composition selon l'une des revendications 9 ou 10 destinée à une utilisation dans une thérapie hyperthermique.

# FIG. 1

INVENTION
Fe CONCENTRATION
1. 25% ( w/v )

✳ CONTROL IS DIFFICULT

COMPARISON (ORDINARY PRODUCT)
Fe CONCENTRATION
5.0% (w/v)

COMPARISON (ORDINARY PRODUCT)
Fe CONCENTRATION
1.25% ( w/v )

TEMPERATURE OF A SOLUTION

°C

43

40

37

I

III

II

1    2    3    4    5  min.

INDUCTION HEATING TIME

EP 0 444 194 B1